# FASCICULE DE BREVET EUROPEEN

(11) **EP 0 624 585 B1**
(45) Date de publication et mention de la délivrance du brevet: **18.08.1999**
(21) Numéro de dépôt: 94401018.0
(22) Date de dépôt: 09.05.1994
(51) Int. Cl.: C07D 403/12, A61K 31/505

(54) **Procédé de préparation de 2-(4-(4-(4-chloro-1-pyrazolyl)butyl) 1-pipérazinyl)pyrimidine**
Verfahren zur Herstellung von 2-(4-(4-(4-chloro-1-pyrazolyl)Butyl)1-Piperazinyl)Pyrimidin
Process for the preparation of 2-(4-(4-(4-chloro-1-pyrazolyl)butyl)1-piperazinyl)pyrimidine

(30) Priorité: 10.05.1993 FR 9305586
(43) Date de publication de la demande: 17.11.1994
(73) Titulaire: LABORATORIOS DEL DR. ESTEVE, S.A., 08026 Barcelona (ES)
(72) Inventeur: Merce-Vidal, Ramon, E-08014 Barcelone (ES); Frigola-Constansa, Jordi, E-08013 Barcelone (ES)
(74) Mandataire: Ahner, Francis

(56) Documents cités:
- EP-A- 0 382 637
- EP-A- 0 497 658

## Description

La présente invention concerne un nouveau procédé de préparation de 2-{4-[4-(4-chloro-1-pyrazolyl)butyl]1-pipérazinyl}pyrimidine (Lesopitron) de formule I :

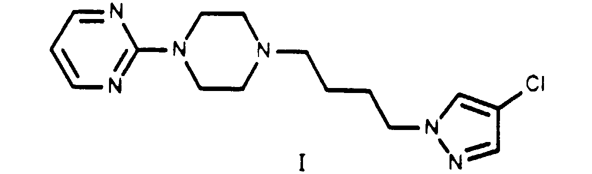

Le Lesopitron (E-4424) est un composé à activité pharmacologique sur le système nerveux central qui présente une activité anxiolitique et tranquilisante (EP-A 382 637). En outre on peut l'utiliser dans le traitement d'autres maladies du comportement (EP-A 429 360 et EP-A 497 658)

Le laboratoire demandeur a décrit différentes synthèses du Lesopitron (EP-A 382 637 et EP-A 502 786). En général, toutes ces synthèses se fondent sur les trois fragments suivants du produit final :
a) pyrimidylpipérazine (II),
b) chaîne aliphatique à quatre atomes de carbone disubstituée (III), et
c) 4-chloropyrazole(IV).

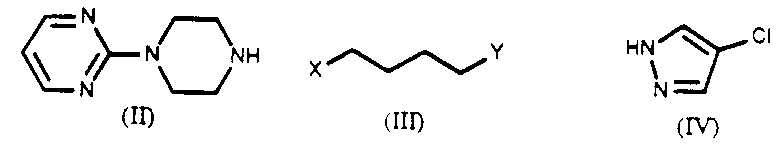

Dans la formule générale (III), X et Y représentent un groupe partant comme les groupes tosyloxy ou mésyloxy ou un atome d'halogène.

Ce type de synthèse se réalise principalement en deux étapes :
- condensation de la chaîne carbonée (III), avec un des fragments (II) ou (IV), puis
- condensation du produit obtenu avec la seconde unité (IV) ou (II), respectivement.

La présente invention concerne un nouveau procédé de préparation de 2-{4-[4-(4-chloro-1-pyrazolyl)butyl]1-pipérazinyl}pyrimidine (Lesopitron) (I), dans lequel la condensation des trois fragments ci-dessus est effectuée en une seule étape.

Ce procédé permet d'obtenir le Lesopitron avec des rendements très élevés et améliore la synthèse du point de vue industriel.
Conformément à la présente invention, le procédé de préparation du composé de formule I est caractérisé en ce qu'on effectue en une seule étape dans un solvant choisi parmi le groupe comprenant, le diméthylformamide, le diméthylsufoxyde, l'isopropanol, le tert-butanol, le toluène ou le xylène, la réaction entre la 2-(1-pipérazinyl)pyrimidine, le 4-chloropyrazole et la chaîne carbonée de formule (III) 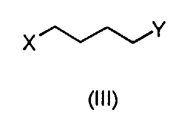 dans laquelle X et Y identiques ou différents, représentent un groupe partant choisi parmi l'iode, le brome, le chlore, un groupe mésyloxy ou un groupe tosyloxy, ladite réaction étant conduite en présence d'une base appropriée organique ou inorganique.

D'une manière préférentielle, dans le composé de formule III, les radicaux X et Y sont identiques.

La réaction est de préférence conduite en présence d'un carbonate de métal alcalin.

La température réactionnelle est comprise entre 80°C et 180°C et la pression est de préférence comprise entre 1,013.10⁵ et 5,065.10⁵ bar (1 atm et 5 atm). On peut conduire la réaction sans catalyseur ou en présence d'un ou plusieurs agent(s) de transfert de phase, comme les sels de tétrabutylammonium. Le temps de réaction est avantageusement compris entre 1 et 24 heures.

En opérant selon l'invention, on obtient le composé I pur et avec des rendements très élevés. En outre, le procédé selon l'invention simplifie sensiblement la mise au point industrielle, entraînant une économie de temps, d'énergie et de produits nécessaires à la réaction.

D'autres caractéristiques apparaîtront à la lumière de l'exemple ci-après.

### Exemple

### Préparation de 2-{4-[4-(4-chloro-1-pyrazolyl)butyl]1 pipérazinyl}pyrimidine.

A un mélange de 2-(1-pipérazinyl)pyrimidine (32,8 g, 0,2 mole), 1,4-dibromobutane (47,5 g; 0,22 mole) et K₂CO₃ (69 g ; 0,5 mole) dans 400 ml de diméthylformamide on ajoute du 4-chloropyrazole (20,5 g, 0,2 mole) et on maintient au reflux pendant 17 heures. On filtre le mélange réactionnel à chaud et on le fait évaporer jusqu'à siccité. On le dissout dans HCl, on lave avec CHCl₃, on alcalinise avec NaOH dilué et on extrait en milieu basique avec CHCl₃. On fait alors sécher la phase organique puis on la fait évaporer jusqu'à siccité, et l'on obtient 61 g (95%) de 2-4-4(4-chloro-1-pyrazolyl)butyl-1-pipérazinyl-pyrimidine.

### Données spectroscopiques :

- IR (film); 2843, 1586, 1547, 1358, 983 cm⁻¹.
- RMN-¹H(δ, CDCl₃) : 8,25 (d, 2H, j=4,7 Hz); 7,39 (s, 1H); 7,35 (s, 1H); 6,44 (t, 1H, j=4,7Hz); 4,0 (t, 2H, j=6,8Hz); 3,80 (m,4H); 2,43 (m, 6H); 1,90 (m, 2H); 1,52 (m, 2H).

Conformément au procédé selon l'invention, les trois composés de départ des formules II, III et IV sont de préférence utilisées dans des proportions approximativement équimolaires.

En outre, si l'on rajoute une base dans dans le milieu de réaction, celle-ci devrait être introduite dans une proportion molaire base/pyrimidinylpipérazine de formule II comprise entre deux et trois.

## Revendications

1. Procédé de préparation de 2-{4-[4-(4-chloro-1-pyrazolyl)butyl]1-pipérazinyl}pyrimidine (Lesopitron) de formule I : 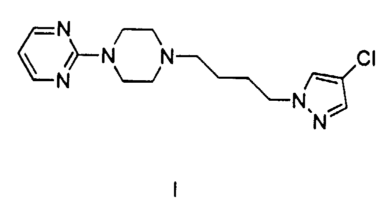 caractérisé en ce qu'on effectue en une seule étape dans un solvant choisi parmi le groupe comprenant, le diméthylformamide, le diméthylsufoxyde, l'isopropanol, le tert-butanol, le toluène ou le xylène, la réaction entre la 2-(1-pipérazinyl)pyrimidine, le 4-chloropyrazole et la chaîne carbonée de formule (III) 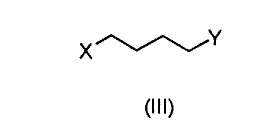 dans laquelle X et Y identiques ou différents, représentent un groupe partant choisi parmi l'iode, le brome, le chlore, un groupe mésyloxy ou un groupe tosyloxy, ladite réaction étant conduite en présence d'une base appropriée organique ou inorganique.

2. Procédé selon la revendication 1, caractérisé en ce que ladite base est un carbonate de métal alcalin.

3. Procédé selon l'une des revendications 1 et 2, caractérisé en ce que dans le composé de formule III, les radicaux X et Y sont identiques.

4. Procédé selon l'une des revendications 1 à 3, caractérisé en ce que la température réactionnelle est comprise entre 80°C et 180°C.

5. Procédé selon l'une des revendications 1 à 4, caractérisé en ce que la pression réactionnelle est comprise entre 1,013.10⁵ et 5,065.10⁵ bar (1 et 5 atm).

6. Procédé selon l'une des revendications 1 à 5, caractérisé en ce que la réaction est conduite en présence d'un ou plusieurs agents de transfert de phase, comme les sels de tétrabutylammonium.

7. Procédé selon l'une des revendications 1 à 6, caractérisé en ce que le temps de réaction est compris entre 1 et 24 heures.

## Patentansprüche

1. Verfahren zur Herstellung von 2-{4-[4-(4-Chlor-1-pyrazolyl)butyl]1-piperazinyl}pyrimidin (Lesopitron) der Formel I: 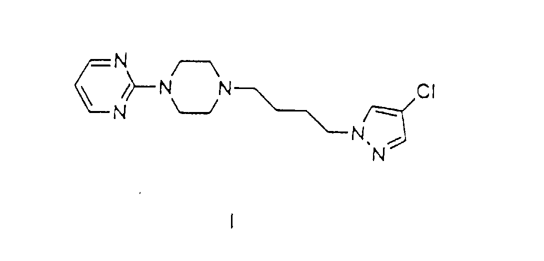 dadurch gekennzeichnet, daß man in einer einzigen Stufe in einem Lösungsmittel, ausgewählt aus der Gruppe von Dimethylformamid, Dimethylsulfoxid, Isopropanol, tert-Butanol, Toluol oder Xylol die Reaktion von 2-(1-Piperazinyl)pyrimidin, 4-Chlorpyrazol und der Kohlenstoffkette der Formel (III) 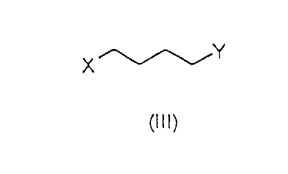 worin X und Y gleich oder verschieden sind und eine austretende Gruppe ausgewählt aus Iod, Brom, Chlor, einer Mesyloxygruppe oder einer Tosyloxygruppe darstellen, durchführt, wobei die Reaktion in Anwesenheit einer geeigneten organischen oder anorganischen Base durchgeführt wird.

2. Verfahren nach Anspruch 1, dadurch gekennzeichnet, daß die Base ein Alkalimetallcarbonat ist.

3. Verfahren nach einem der Ansprüche 1 und 2, dadurch gekennzeichnet, daß die Reste X und Y in der Verbindung der Formel III identisch sind.

4. Verfahren nach einem der Ansprüche 1 bis 3, dadurch gekennzeichnet, daß die Reaktionstemperatur zwischen 80°C und 180°C liegt.

5. Verfahren nach einem der Ansprüche 1 bis 4, dadurch gekennzeichnet, daß der Reaktionsdruck zwischen 1,013.10⁵ und 5,065.10⁵ bar (1 und 5 atm) liegt.

6. Verfahren nach einem der Ansprüche 1 bis 5, dadurch gekennzeichnet, daß die Reaktion in Anwesenheit von einem oder mehreren Phasenübertragungsmitteln, wie die Tetrabutylammoniumsalze, durchgeführt wird.

7. Verfahren nach einem der Ansprüche 1 bis 6, dadurch gekennzeichnet, daß die Reaktionszeit zwischen 1 und 24 Stunden liegt.

## Claims

1. Process for the preparation of 2-{4-[4-(4-chloro-1-pyrazolyl)butyl]-1-piperazinyl}pyrimidine (Lesopitron) of formula I: 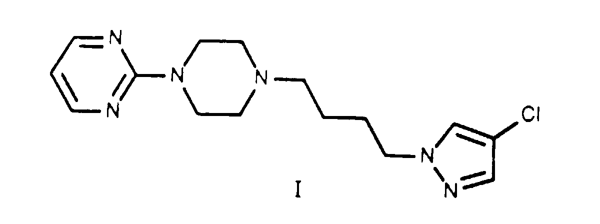 characterized in that the reaction between 2-(1-piperazinyl)pyrimidine, 4-chloropyrazole and the carbon chain of formula (III) 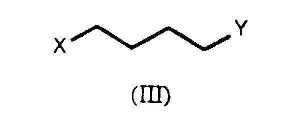 in which X and Y, which may be identical or different, represent a leaving group chosen from iodine, bromine or chlorine, a mesyloxy group or a tosyloxy group, is carried out in a single step in a solvent chosen from the group comprising dimethylformamide, dimethyl sulphoxide, isopropanol, tert-butanol, toluene or xylene, the said reaction being carried out in the presence of an appropriate organic or inorganic base.

2. Process according to Claim 1, characterized in that the said base is an alkali metal carbonate.

3. Process according to either of Claims 1 and 2, characterized in that, in the compound of formula III, the radicals X and Y are identical.

4. Process according to one of Claims 1 to 3, characterized in that the reaction temperature is between 80°C and 180°C.

5. Process according to one of Claims 1 to 4, characterized in that the reaction pressure is between 1.013x10⁵ and 5.065x10⁵ bars (1 and 5 atm).

6. Process according to one of Claims 1 to 5, characterized in that the reaction is carried out in the presence of one or more phase transfer agents, such as tetrabutylammonium salts.

7. Process according to one of Claims 1 to 6, characterized in that the reaction time is between 1 and 24 hours.
